(19) **Europäisches Patentamt**
**European Patent Office**
**Office européen des brevets**

(11) **EP 1 231 840 B1**

(12) **EUROPEAN PATENT SPECIFICATION**

(45) Date of publication and mention
of the grant of the patent:
**22.07.2009 Bulletin 2009/30**

(51) Int Cl.:
*A01N 63/00* (2006.01)  *A61K 39/00* (2006.01)
*C12N 5/06* (2006.01)  *C12N 5/02* (2006.01)
*C12N 5/08* (2006.01)  *A61K 38/00* (2006.01)
*A01N 65/00* (2009.01)

(21) Application number: **00987995.8**

(22) Date of filing: **22.11.2000**

(86) International application number:
**PCT/US2000/032322**

(87) International publication number:
**WO 2001/037671 (31.05.2001 Gazette 2001/22)**

(54) **ANTI-ANGIOGENIC CELLULAR AGENT FOR CANCER THERAPY**

ANTI-ANGIOGENER ZELLULÄRER WIRKSTOFF ZUR KREBSBEHANLDUNG

AGENT CELLULAIRE ANTI-ANGIOGENIQUE DESTINE AU TRAITEMENT DU CANCER

(84) Designated Contracting States:
**AT BE CH CY DE DK ES FI FR GB GR IE IT LI LU MC NL PT SE TR**

(30) Priority: **24.11.1999 US 167513 P**

(43) Date of publication of application:
**21.08.2002 Bulletin 2002/34**

(73) Proprietor: **Hope, Ernest G.**
**Durham, NC 27705 (US)**

(72) Inventor: **Hope, Ernest G.**
**Durham, NC 27705 (US)**

(74) Representative: **König, Gregor Sebastian**
**König Szynka Tilmann von Renesse**
**Patentanwälte Partnerschaft**
**Postfach 11 09 46**
**40509 Düsseldorf (DE)**

(56) References cited:
**WO-A-00/10603**    **WO-A-99/46365**
**US-A- 5 660 827**

• **DATABASE DISSABS Order Number AAI9958115, Vol. 61, No. 1b, p. 178 1999, HOPE, ERNEST G.: "Heat shock protein 47 mediated response modulation on noMHC I restricted cytotoxic T lymphocytes" XP002285609 -& E.G. HOPE: "PhD-thesis: Heat shock protein 47 mediated response modulation on non-MHC I restricted cytotoxic T lymphocytes" June 1999 (1999-06), STANDFORD UNIVERSITY , XP002285610 * the whole document ***

• **HOPE E G ET AL: "RECOMBINANT HUHSP47 IS A NOVEL SELECTIVE IMMUNE-SUPPRESSANT AGENT FOR ADOPTIVE IMMUNOTHERAPY (AI): IT PROTECTS ENDOTHELIAL CELLS FROM DAMAGE BY AI CELLS BUT DOES NOT CAUSE REDUCTION OF TUMOR SURVEILLANCE ACTIVITY" BLOOD, W.B. SAUNDERS, PHILADELPHIA, VA, US, vol. 92, no. 10SP01PT01/2, 15 November 1998 (1998-11-15), page 341A, XP009027959 ISSN: 0006-4971**

• **SCHMIDT-WOLF I G H ET AL: "Phase I clinical study applying autologous immunological effector cells transfected with the interleukin-2 gene in patients with metastatic renal cancer, colorectal cancer and lymphoma" BRITISH JOURNAL OF CANCER, vol. 81, no. 6, November 1999 (1999-11), pages 1009-1016, XP002285306 ISSN: 0007-0920**

• **SCHMIDT-WOLF G D ET AL: "Activated T cells and cytokine-induced CD3+CD56+ killer cells" ANNALS OF HEMATOLOGY, BERLIN, DE, vol. 74, no. 2, 1997, pages 51-56, XP002176407 ISSN: 0939-5555**

EP 1 231 840 B1

- MEHTA BELA A ET AL: "Two pathways of exocytosis of cytoplasmic granule contents and target cell killing by cytokine-induced CD3+CD56+ killer cells" BLOOD, vol. 86, no. 9, 1995, pages 3493-3499, XP002285307 ISSN: 0006-4971
- KANEKO T ET AL: "CYTOTOXICITY OF CYTOKINE-INDUCED KILLER CELLS COATED WITH BISPECIFIC ANTIBODY AGAINST ACUTE MYELOID LEUKEMIA CELLS" LEUKEMIA AND LYMPHOMA, HARWOOD ACADEMIC PUBLISHERS, CHUR, CH, vol. 14, 1994, pages 219-229, XP002048525 ISSN: 1042-8194
- LEFTEROVA P ET AL: "Targeting of CD3+CD56+ human immunologic effector cells against CD19+ leukemia and lymphoma cells by the use of anti-CD3 monoclonal antibody" BLOOD, vol. 92, no. 10 SUPPL. 1 PART 1-2, 15 November 1998 (1998-11-15), page 246A, XP009032417 40th Annual Meeting of the American Society of Hematology;Miami Beach, Florida, USA; December 4-8, 1998 ISSN: 0006-4971
- HU Z ET AL: "TARGETING TUMOR VASCULATURE ENDOTHELIAL CELLS AND TUMOR CELLS FOR IMMUNOTHERAPY OF HUMAN MELANOMA IN A MOUSE XENOGRAFT MODEL" PROCEEDINGS OF THE NATIONAL ACADEMY OF SCIENCES OF USA, NATIONAL ACADEMY OF SCIENCE. WASHINGTON, US, vol. 96, February 1999 (1999-02), pages 8161-8166, XP002931078 ISSN: 0027-8424
- LU ET AL.: 'A novel population of expanded human CD3+CD56+ cells derived from T cells with potent in vivo antitumor activity in mice with severe combined immunodeficiency' J. IMMUNOL. vol. 183, 1994, pages 1687 - 1696, XP002938495
- ZOLL ET AL.: 'Generation of cytokine induced killer cells using exogenous interleukin-2, -7, or -12' CANCER IMMUNOL. IMMUNOTHER. vol. 47, 1998, pages 221 - 226, XP002938494
- HOYLE ET AL.: 'Expansion of Philadelphia chromosome-negative CD3+CD56+ cytotoxic cells from chronic myeloid leukemia patients: In vitro and invivo efficacy in severe combined immunodeficiency disease mice' BLOOD vol. 92, no. 9, 01 November 1998, pages 3318 - 3327, XP002938493

**Description**

Field of the Invention

The invention relates to methods of using immune system cells in the treatment of cellular proliferative disease and cancer.

Background of the Invention

Adoptively transferred cellular immunity constitutes a major factor controlling relapse in some cancer patients undergoing allogeneic hematopoietic cell transplantation. Immune-mediated benefits of bone marrow transplantation (BMT) were first described as the "graft-versus-leukemia" (GvL) effect (Sullivan et al. (1989) Blood 73:1720; Weiden et al. (1979) N. Engl. J. Med 300:1068). Both natural killer (NK) cells and T cell subsets contribute to GvL (Antin (1993) Blood 82:2273; Hauch et al. (1990) Blood 75:2250). Depletion of T cells from the donor hematopoietic cell population results in enhanced relapse rates following BMT (Martin et al. (1985) Blood 66:664). Infusion of donor lymphocytes into patients who have relapsed following allogeneic BMT results in a significant percentage of patients achieving durable complete remissions (Kolb et al. (1995) Blood 86:2041; Porter et al. (1994) N. Engl. J. Med. 330:100).

Several distinct T and NK cell populations have been expanded *ex vivo* and utilized as effector cells for adoptive immune therapy. These populations include lymphokine activated killer (LAK) cells, tumor-infiltrating lymphocytes (TIL), and cytokine induced killer (CIK) cells.

LAK cells are interleukin-2 (IL-2) dependent short-term culture products derived from NK cells. LAK cells recognize a broad array of tumor cell targets and autologous tumor cells in vitro. However, their *in vivo* efficacy is limited by their limited proliferative potential and the requirement for the co-application of IL-2 (Rosenberg et al. (1985) J. Exp. Med. 161:1169). Systemic administration of IL-2 is associated with considerable toxicities, including massive endothelial destruction (Siegel et al. (1991) J. Clin. Oncol. 9:694). LAK cell therapy in patients has caused significant morbidity due to vascular leak syndrome associated with endothelial destruction (VLS) (Kotasek et al. (1988) Cancer Res 48:5528).

TILs have greater tumor antigen specificity than LAK cells. However, TILs must be isolated from a surgical specimen of the patient and their generation tends to be cumbersome and yields low cell numbers. Specific tumor antigens are rarely available to ensure sufficient *in vitro* expansion of TILs for either successive treatments or dose escalations (Rosenberg et al. (1988) N. Engl. J. Med. 319:1676).

CIK cells are generated from peripheral blood mononuclear cells (PBMC) by culture in the presence of IFN-$\gamma$, OKT-3, and IL-2. CIK cell cultures rapidly expand in the absence of target cells, resulting in the preferential expansion of the otherwise rare population of CD3$^+$CD16$^-$CD56$^+$ $\alpha/\beta$T cells. CIK cells have superior *in vivo* GvL activity as compared to LAK cells and are capable of purging SCID mice from lethal hematopoietic tumor burden without the need for the systemic administration of IL-2 (Lu et al. (1994) J.Immunol. 153:1687).

Summary of the Invention

The invention features a method, uses and compositions as defined in the dependent claims.

Anergic T cells of various lineages can, under certain conditions, be stimulated with cytokines to activate effector cell mechanisms that permit the cells to selectively attack tumor vasculature. Without being bound by any particular theory, it appears that this effector cell function is an evolutionarily archaic one that is not limited to alpha/beta T cells or gamma/delta T cells. The desired effector cell function is present in at least a subclass of cells known as CIK cells. In addition, the effector cell function may also be present on certain cells that do not express a T cell receptor. For example, the function may be present in a subclass of NK cells.

The immune cells of the disclosure are referred to collectively as ex vivo expanded anti-angiogenic T cells ("EAT cells"). The immune cells of the disclosure can be derived from the cells of the patient being treated or another subject, thus they can be allogeneic or autologous, and are expanded ex vivo to generate cells with the desired properties and in sufficient quantity.

The selectivity of EAT cells is believed to be regulated, at least in part, by one or more of Hsp47, Hsp16, Hsp27, Hsp32, and possibly other heat shock proteins such as Hsp65, Hsp70, Hsp72, Hsp94, Hsp 90, Hsp96, and Hsp larger than 100kD and smaller than 60 kD. Hsp47 belongs to a family of recognized immune modulators that include, but are not limited to, Hsp47, HLA-A subtypes, and IL-12 receptors. Thus, EAT cells can bear (i.e., express) a cell surface receptor which binds to Hsp47, an HLA molecule (e.g., HLA-A or an HLA-A subtype), IL-12 receptor or one of the aforementioned Hsp proteins.

EAT cells can be isolated from a population of activated killer cells by positive selection of cells that express receptors as outlined above, or by negative selection that destroys or discards cells that do not have such receptors or show reactivity against normal vasculature. For example, in vitro cultured endothelial cells that are either pre-incubated to functional saturation with a functional member of the Hsp47 family of immune modulators or that have been genetically transformed to secrete Hsp47 or a related protein can be used to select EAT cells having desirable properties. Use of a peptide corresponding to shared homologies between members of the Hsp47 family of immune modulators can substitute for the expression or use of full length of partial polypeptides of this immune modulator. Immune cells that attack endothelial cells in the presence of a functionally sufficient amount of Hsp47 (i.e., an amount that would inhibit killing by an equivalent cell dose of CIK cells, e.g., using an assay described below) are eliminated by negative selection. Positive selection can be carried out after staining with Hsp47 or functional substitute and subsequent FACS sorting of positively stained cells, or by panning using immobilized (i.e., on magnetic beads, a column or some other solid support) Hsp47 or a functional substitute. Similar approaches can be used for negative selection. In some circumstances, no purification of EAT cells from the population in which they are generated is necessary.

Unlike certain cells used for cellular immunotherapy of cancer, EAT cells do not cause vascular leak syndrome (VLS), a very serious, often life threatening side effect of cellular immunotherapy. VLS arises from the damage caused to endothelial cells in the normal vasculature caused by certain activated NK cells or activated T cells and may involve activation or interaction with other immune system cells. Due the very serious consequences of VLS, cellular immunotherapy is generally considered appropriate only for patients suffering from terminal renal cancer or terminal melanoma. In contrast, because the risk of VLS associated with EAT cell administration is minimal, EAT cells can be used for treatment of vascularized cancers generally, including non-malignant cancers, early stages of a cancer, cancer of any grade and stage and even non-life threatening disorders that are associated with inappropriate vascularization (e.g., endometriosis).

Many types of EAT cells directly attack tumor cells in addition to attacking tumor vasculature. Thus, such EAT cells can be particularly effective for the treatment of cancer. Because EAT cells attack tumor vasculature they can be therapeutically effective for treatment of vascularized cancer even if they do not substantially kill the tumor cells themselves either in vitro or in vivo. Thus, cells that do not kill cells of a particular cancer cell line in vitro can nonetheless be effective therapeutically for treating that cancer because they can attack tumor vasculature.

In some cases a particular method for generating a type of EAT cell will actually generate a variety of cell types, e.g., one type capable of attacking tumor vasculature and another type capable of attacking tumor cells themselves. In some circumstances the various cell types can be used together. In other cases it can be desirable to generate pure populations of each of the cells types, e.g., by FACS or some other suitable method. It can also be desirable to generate a clonal population of a cell generated *ex vivo.*

EAT cells, including CIK cells can be used without the co-administration of a cytokine, e.g., IL-2. EAT cells, including CIK cells can be administered as cells that are fully capable of replication, i.e., they are preferably not lethally irradiated. In addition, EAT cells are preferably administered without co-administration of a cytokine, e.g., an interleukin such as IL-1, IL-2, IL-7, IL-12, or IL-15. In addition, the cells are preferably not stably transfected with a nucleic acid molecule encoding a cytokine, e.g., an interleukin such as IL-1, IL-2, IL-7, IL-12, or IL-15, or any other nucleic acid molecule. However, the cells can be transiently or stably transfected with a nucleic acid molecule encoding a cell surface marker. In a typical application of bulk, unfractionated cells, the total cell dosage is preferably least $10^8$, $10^9$, $10^{10}$, $10^{11}$, $10^{12}$, $10^{13}$, $10^{14}$, or $10^{15}$ (including non-functional cells or non-EAT cells) cells per patient or per treatment. Because the cells are safer than other immune cell therapies, the upper limit on the dosage is very higher, perhaps exceeding $10^{15}$. Cells can be titrated to determine the number of lytic units for a selected tumor or anti-angiogenic target. The dosage can then be adjusted accordingly. In some circumstances much lower does can be used ($10^4$, $10^5$, $10^6$, $10^7$). The cell dose per administration can be greater than $1.25 \times 10^{10}$ or $2.5 \times 10^{10}$ for patients suffering from solid tumors and at least $2 \times 10^9$ cells or $4 \times 10^9$ for patients suffering from hematopoietic tumors.

Preferably the EAT cells, particularly CIK cells, are grown with agitation or aeration of the media rather than in static cultures. CIK cells grown with agitation or aeration can be grown to a higher cell density and are more effective than cells grown in static culture. Thus, the cells can be grown in closed culture systems, spinner reactors, perfusion cultures, hollow fiber culture, spheroid culture, membrane enclosed culture (suspended in aerated media). Preferably the cells are grown to a density of greater than $3 \times 10^6$ cells/ml, more preferably at least $5 \times 10^6$, $10 \times 10^6$, $15 \times 10^6$, $20 \times 10^6$, $10^7$, $10^8$, $10^9$, $10^{10}$, $10^{11}$, or $10^{12}$ cells/ml. CIK transfected with a nucleic acid molecule other than one encoding IL-2 or IL-7 can be used to treat patients.

Many other types of cells can be EAT cells, including: subclasses of T cells, subclasses of NK cells, and subclasses of cells generated by stimulation of dendritic cells. Preferred cells utilize the lack of a non-kill signal to recognize tumor cells and to recognize tumor vasculature components (e.g., tumor vasculature endothelial cells, smooth muscle cells, fibroblasts, and other tumor vasculature components). In addition, preferred cells are non-classical MHC restricted. In some cases, the cells utilize non-kill signal receptor, for example, a receptor for Hsp 47 or another extracellular matrix component.

The cells can be combined with a bi-specific antibody or multi-specific antibody which binds to the EAT cell and to the target cell. Molema et al. 2000 J. Controlled Release 64:229-39. A bi-valent or multi-valent antibody can also bind to a molecule to n be delivered include: a toxin, a radioactive molecule, an immune modulator, and a tracer. A mono-valent antibody can be linked to a molecule to be delivered to the target cell.

Because EAT cells are not associated with a significant VLS, they can be used to treat far less severely ill patients than patients treated with traditional immunotherapy, e.g., patients treated with activated NK cells, and they can be administered a far higher active cell dose. Moreover, patients can be treated more cost effectively in a wholly pr partially out patient context. The patient can be treated in a out patient setting, can be treated until the tumor shrinks to 1%. 2%, 5%, 10%, 20%, 30%, 40%, 50%, 60%, or 70% of its original size or until it disappears completely. Thus, the cells can be used in an intent to cure context. EAT cells can also be used as a preventative or prophylactic therapy in healthy patients or in cancer survivors.

EAT cells can be used to treat, for example: hematopoietic cancer relapse subsequent to bone marrow transplant, any carcinoma, any sarcoma, liquid tumors (e.g., multiple myeloma, Waldenstroms' (IgM) gammopathy, Bergers (IgA), CNS lymphoma (e.g., associated with AIDS), gonadal lymphomas and leukemias, mantle cell lymphomas, vascularized stages of leukemias (bone marrow) and lymphomas (in the lymph nodes), and any other leukemia or lymphoma, including low grade leukemias and lymphomas), solid tumors (i.e., vascularized tumors, including angiosarcomas, Kaposi's sarcoma, mesothelioma, Ewing's Sarcoma, choriocarcinoma, ascitis tumors (e.g., ovarian cancer especially with peritoneal implants), gonadal cancers (including ovarial and cervical), airway cancers (small cell lung, lung, and bronchial), gastrointestinal cancers (pancreatic, intestinal, colon, rectal, small intestinal, polyposis, gall duct, stomach), esophageal cancer, Barrett's esophagus cancer, oral cancer, parotid cancer, nasopharyngeal cancer, thyroid cancer, CNS cancers (glial, neuroblastoma multiforme, neuromas, meningiomas, astrocytomas, any other pediatric or adult CNS cancer), urogenital cancers (bladder, renal, adrenal, prostate), skin cancers (melanoma nodular, invasive, superficial spreading MF, squamous cell, lip) bone and connective tissue cancers (breast, bone, chondromas, leiomyomas, Wilm's tumor, retinoblastoma), and any other solid carcinoma or sarcoma of pediatric or adult patients. EAT cells can also be used to treat non-malignant diseases that are associated with neovacularization, for example: AV malformation, endometriosis, and any state of increased vascularity that is not considered to reflect perfect health, including, but not limited to auto-immune diseases such as psoriasis or rheumatoid arthritis. They may also be useful for treatment of any parasitic, viral or fungal infections with neo-vascularization, such as, but not limited to echinococcus granulosus and sarcoid. EAT cells can be used to treat a wide variety of disorders which would be ameliorated if associated neovascularization or associated vascularization were reduced.

EAT cells selectively damage tumor associated vasculature compared to normal vasculature. This selectivity can be measured by comparing the killing of cultured HUVEC in the absence and presence of Hsp47. In this assay the concentration of Hsp47 can be equal to that required for CIK cells to exhibit 2-fold, 5-fold, 10-fold or 100-fold selectivity in the assay.

EAT cells selectively damage tumor vasculature, reducing the flow of blood and nutrients to the tumor, arresting or reducing the growth potential of the tumor.

Predictive models can be used to identify patients likely to responder to EAT therapy or anti-angiogenic pharmaceutical therapy prior to, concurrent with, or after subjecting the patients to EAT therapy.

EAT cells can be used in vivo or in vitro to screen for pharmaceutical targets or immunological targets of tumor anti-angiogenesis, including by random peptide library expression, transgene screening of any cells expressing 6-n meric peptides and/or 18-n meric nucleotide coding frames using these transgenes.

EAT cells can be fused to any other cell (hybridoma). EAT cells can be administered with autologous plasma, bovine plasma, or artificial lipid carrying systems. EAT cells can be administered to any mammal, preferably not mice.

Unless otherwise defined, all technical and scientific terms used herein have the same meaning as commonly understood by one of ordinary skill in the art to which this invention belongs. Suitable methods and materials are described below, although methods and materials similar or equivalent to those described herein can also be used in the practice or testing of the present invention.

In case of conflict, the present specification, including definitions, will control. In addition, the materials, methods, and examples are illustrative only and not intended to be limiting.

Other features and advantages of the invention will be apparent from the following detailed description, and from the claims.

Brief Description of the Drawings

Figure 1 depicts the CD3 and CD56 cell surface expression profile of day 21 CIK cells expanded *ex vivo* in a bioreactor.

Figure 2A depicts the cytotoxicity profile of day 21 CIK cells against tumor cell targets (K562, OCI-Ly8, and WM9) and endothelial cell targets (HUVEC).

Figure 2B depicts the cytotoxicity profile of day 21 bioreactor-cultured and standard flask-cultured CIK cells against OCI-Ly8 target cells.

Figure 2C depicts the cytotoxicity profile of day 21 CIK cells against freshly confluent HUVEC cells and HUVEC cells five days post-confluence.

Figure 3 depicts the effects of anti-LFA-1 and anti-ICAM-1 antibodies on CIK cell mediated lysis of OCI-Ly8 cells and HUVEC.

Figure 4 depicts the effects of effector cell treatment with BFA and/or ConA (CMA) on lysis of OCI-Ly8 cells and HUVEC.

Figure 5 depicts the cytotoxicity profiles of CIK cell clones against OCI-Ly8 cells and HUVEC.

Figure 6 depicts a SCID mouse with a human full thickness skin graft.

Figure 7 depicts Brefeldin A induced protection of EC from CIK mediated lysis.

Figure 8 depicts Hsp47 induced protection of EC from CIK mediated lysis.

Figure 9 depicts FACS analysis of CIK cells.

Figure 10 depicts Hsp47 transgene induced protection of EC from CIK mediated lysis.

Detailed Description

Methods of Generating EAT Cells

EAT cells can be generated by a variety of methods. The simplest methods entail incubation of PBLs in media with intermediate to high levels of IL-2. More elaborate methods entail cell selection or cell depletion, stimulation with anti-TcR antibodies and the incubation with cytokines or lymphokines, including incubation with gamma interferon (or IL-12 or IL-15) proceeding stimulation with anti-TcR antibodies. Preferred methods lead to the expansion of cells expressing CD3, CD56 and CD8. Such cells have highly desirable effector function and patient safety characteristics.

EAT, e.g., CIK, cells can be generated from a variety of hematopoietic cell sources, e.g., peripheral blood, bone marrow, spleen, fetal liver, lymph node, thymus, or any tissue in the body containing lymphoid cells. EAT cell precursors can be enriched in *vitro* by a variety of methods prior to cytokine induction. The following exemplary methods can be used either alone or in combination in advance of the cytokine induction. In one example, peripheral blood mononuclear cells are isolated from a peripheral blood sample by density gradient separation, e.g., ficoll density gradient centrifugation. In another example, EAT cell precursors in a hematopoietic cell sample are enriched by selecting for plastic non-adherent cells (to deplete monocytes) and/or nylon wool non-adherent cells (to deplete B cells). In another example, cells displaying a particular cell surface antigenic phenotype are selected, e.g., by panning or by cell sorting. Cells can be selected based upon the presence of a particular antigen, e.g., CD3, CD56, TCR $\alpha/\beta$, or TcR $\gamma/\delta$ or CD8. In addition they and can be selected based upon the absence of a particular antigen, e.g., CD4, CD14, CD16, CD19, or CD20.

Cells enriched according the above methods can be grown in lymphocyte culture media well known to those of skill in the art, e.g., complete RPMI 1640 (GIBCO-BRL/Life Technologies, Grand Island, NY) containing 10% fetal calf serum (FCS), 50 $\mu$m $\beta$-mercapto-ethanol (ME), 100 IU penicillin-G/ml, and 100 IU streptomycin/ml. Methods of CIK cell generation are described in, e.g., Schmidt-Wolf et al. (1999) Br. J. Cancer 81:1009, Hoyle et al. (1998) Blood 92: 3318, and Scheffold et al. (1995) Bone Marrow Transplant. 15:33, the contents of which are herein incorporated by reference.

CIK cells can be generated from a cell sample described above by exposing the cell sample to the combination of a cytokine cocktail and an anti-CD3 antibody, e.g., the OKT3 monoclonal antibody. CIK cells are typically generated by a cytokine cocktail including IL-2 and IFN-$\gamma$. This cocktail can include additional cytokines such as IL-1$\alpha$, IL-7, and/or IL-12. IL-7 and IL-12 can optionally be used either in addition to or in place of IL-2. In one example, 1000 IU/ml recombinant human interferon gamma (rhu $\gamma$-IFN) (Genentech, San Francisco, CA) is added to the cell sample on day 0. On day 1, the cells are stimulated with 25-50 ng/ml of the soluble anti-human CD3 monoclonal antibody OKT-3 and 300 IU/ml of recombinant human interleukin-2 (rhu IL-2) (Cetus/Chiron, Emeryville, CA). Cell cultures are maintained at 37˚C in a humidified atmosphere of 5% $CO_2$ and fresh media containing 300 IU rhu IL-2 is added every 3-7 days. CIK cells generated according to this method can be used, e.g., for in vitro or in vivo methods, following 21 to 30 days in culture.

EAT cells, particularly CIK cells, can be generated using procedures that result in improved biological activity, improved total cell number, and improved individual cell function geared at overcoming prior shortcomings of previous standard of culturing these cells.

EAT, e.g., CIK cells are preferably generated by a means other than in vessels of standing medium at cell densities of between 0.5 x $10^6$ and 20 x $10^6$.

Preferably EAT cells are grown with agitation and/or aeration of the culture, e.g., in "closed" culture system, such as a bioreactor. Thus, they can grown, e.g., with the use of rocking platforms, tumblers, stirred bio-reactors without electrodes, advanced bioreactors, especially with the use of electrodes (e.g. but not limited to $pO_2$, $pCO_2$, RPM, temperature, cell density/OD) inserted into the culture chamber. They are preferably grown with continued feeding and harvesting. In addition it can be useful to employ analogue or digital data collection or processing, turbine agitated bioreactors with or without speed control, perfusions reactors, diffusion reactors, air-lift reactors, hollow fiber reactors, vibro-mixer reactors, reactors with more than one incubation chamber, reactors that allow sterile harvest or sampling of cells, membrane or other enclosures (e.g., enclosures formed of any plastic or synthetic material including infusion bags, fluro-carbon bags, dialysis membranes, especially gas-permeable membranes for static culture) in combination with culture on rocking platforms or any other means of agitating the media within the enclosures. The vessels may be formed of plastic, Teflon®, glass, or steel. The cells can be grown on solid semi-solid carrier support systems. The supports can be static or agitated.

EAT cells can be cultured with non-effector cells, including co-culture with pulsed or unpulsed dendritic cells, T-helper cells, or tumor targets.

EAT cells, e.g., CIK cells can be frozen and shipped. For example, mature EAT or CIK cells or progenitors thereof can be stored by cryopreservation using methods established for the preservation and storage of bone marrow stem cells. The cells can be stored or shipped below -80˚C (between -80˚C and -20˚C, between -20˚C and 0˚C, between 0˚C and 4˚C, or between 4˚C and ambient temperature) with or without computerized deep-freezing algorithms to gradually lower the temperature and with or without the use of anti-freeze additives. The cells can be shipped using bar coding, numerical or alphanumerical identification and tracking systems to identify and deliver products for individual patients. The cells can be frozen in aliquots or in bulk. They may shipped packed and ready to use with or without freezing. They can be kept in continuous culture until additional aliquots are requested. The shipping can be overnight on wet or dry ice, and the cells can be imbedded in methylcellose or alginate calcium. They can be shipped together with a log of all culture parameters for the patient.

DNA, RNA, or polypeptide microarrays can be used in combination with EAT cells to identify targets of any anti-angiogenic cell or pharmaceutical therapy, to generate antigen specific CTLs, to generate targets for antibodies, to generate antibodies, to assess treatment outcomes using genes or gene fragments (e.g.,derived from normal tissue, cancer tissues, or specific angioblasts or tissue specific endothelial preparations). Arrays can include synthetic gene fragments designed to model 3-dimensional shapes or interaction surfaces in order to select/identify receptor or counter-receptor families in EAT cells as a basis for further purification and selection strategies. EAT cells can be analyzed using a microarray for the purpose of data collection or analysis from clinical EAT trials, to assess vascular selectivity or for any other purpose.

Non-MHC Class I Restricted Cytotoxicity Mediated by CIK Cells

CIK cells cultured *in vitro* share some phenotypic and functional properties with T cells and activated NK cells. CIK cells exhibit significant cytotoxicity against a variety of hematopoietic and solid tumor targets as well as endothelial cells cultured *in vitro* (see examples). All target cells examined herein expressed normal levels of MHC class I. However, the anti-MHC class I monoclonal antibody W6/32 did not inhibit CIK cell-mediated target lysis. The fact that this antibody, which prevents the interaction between a T cell receptor and an MHC class I molecule, cannot block either tumor or endothelial cell lysis demonstrates that CIK cells represent non-classical MHC class I restricted cytotoxic T-cells and that they do not lyse targets via allo-recognition of target cell MHC class I molecules. This is the basis for the use of EAT/CIK cells in allogenic settings without fear of causing serious or fatal graft versus host disease.

In terms of evolutionary immunology, cell-mediated vascular attack is an ancient defense mechanism that does not appear to be mediated solely by NK cells. In colony forming tunicates, non-MHC class I restricted vascular attack is one of the principal mechanisms of allograft rejection when distinct colonies come into contact. The anti-angiogenic activity of CIK cells described herein may reflect the activation of an ancient self-nonself recognition system.

High dose TH$_1$ type hormonal stimulation (e.g., IFN-$\gamma$ and IL-2) of peripheral blood lymphocytes leads to rapid expansion of CD3+CD56+ effector cells in the absence of target cells. Prior to this stimulation, the progenitor cell population does not display significant non-MHC class I restricted cytotoxicity against either tumor or cultured EC targets. CIK cells can be generated from purified T-cell sources, and generally display activation and differentiation markers (e.g., CD45RA). The plurality of surface markers expressed on CIK clones and bulk cultures makes unlikely the existence of a specific CIK T-cell lineage having a distinct precursor.

The *in vitro* CIK culture conditions comprising high dose IL-2 stimulation may rescue previously anergic T cells and NK cells, allowing them to resume cell division and to express new, non-autoreactive target specificities (Madrenas et al. (1996) Proc. Natl. Acad. Sci. U S A 93:9736). CIK cell culture conditions, which provide high dose IL-2 stimulation as well as mitogenic stimulation, may rescue a heterogeneous pool of previously anergic peripheral blood lymphocytes. Mature CIK cells maintain their expression patterns of non-functional receptors and additionally acquire the *de novo* expression of effector molecules involved in newly gained anti-tumor functions. This model not only explains the observed heterogeneity of cell surface markers present on equally functional CIK cell populations, but also addresses the loss of function associated with CAMs, TcR, CD4, and CD8.

Distinct Mechanisms of CIK and EAT Cell Lysis of EC Targets and Tumor Targets

CTL target cell lysis requires both a specific TcR-MHC class I interaction as well as the non-specific interaction between the co-stimulatory molecules ICAM-1 and LFA-1. T cell co-stimulation through ICAM-1/LFA-1 ligation involves myosin motor protein mediated accumulation of co-stimulatory signaling molecules in the T cell/tumor target interface, leading to an amplified and prolonged signaling process (Wulfing et al. (1998) Science 282:2266; Wulfing et al. (1998) Proc. Natl. Acad. Sci. U S A 95:6302). NK cells also utilize the ICAM-1/LFA-1 interaction when killing target cells that lack the proper MHC class I-mediated "no-kill" signal.

Distinct mechanisms of CIK cell recognition of tumor cells and EC cells was apparent in the antibody blocking studies described herein. The interaction between ICAM-1 on tumor targets and LFA-1 on CIK effector cells was necessary for the efficient lysis of the tumor targets. However, the ICAM-1/LFA-1 interaction did not appear to be necessary for CIK cell-mediated lysis of EC cells. These data suggest that CIK/EAT cell-mediated cytotoxicity against tumor targets and EC targets is mediated by distinct mechanisms.

BFA and conA treatments of CIK cells revealed further differences between the lysis of tumor targets and EC targets. These treatments were used to estimate the contribution of perforin/granzyme (conA treatment) an Fas-based mechanisms (BFA treatment) in CIK cell-mediated target cell lysis. EC lysis was virtually entirely perforin and granzyme based, whereas CIK cell-mediated lysis of tumor targets utilized both pathways, with the Fas-pathway dominating lysis of hematopoietic targets.

*In Vitro* Lysis of EC by EAT Cells Not "Innocent Bystander Cytotoxicity"

Cultured vascular endothelial cells secrete fibronectin and laminin into both the extracellular matrix (ECM) and the culture medium (Vlodavsky et al. (1981) Nature 289:304). The control of cell growth and differentiation depends on both cell-cell and cell-substrate interactions which are in part mediated by plasma membrane receptors for ECM components such as fibronectin, laminin and collagen. Cell signaling is at least partially dependent on the lateral mobility of such ECM receptors. This mobility can vary significantly according to the substrate upon which cells adhere (collagen, fibronectin or a natural basement membrane) and on the topography of the cell (basal versus apical plasma membrane) (Nakache et al. (1985) Nature 317:75).

Standard endothelial cell culture conditions typically deprive EC of cell-cell contact, as cell passaging occurs before monolayer confluency is reached. The repeated passaging of EC with trypsin removes ECM proteins from the culture and following trypsinization cells are deposited on standard cultured or heat denatured collagen matrices. $^{51}$Cr release cytotoxicity assays using EC as targets are typically carried out within 24 hours of an EC monolayer reaching confluency.

As described herein, the susceptibility of EC monolayers to lysis by CIK cells *in vitro* is inversely correlated with time the EC are cultured after reaching confluency. This observation suggests a protective role for extracellular matrix factors that are deposited in a time dependent fashion after the trypsinization and passaging of EC *in vitro.*

The destruction of monolayer cultured EC in by CIK cells *in vitro* is not predictive of VLS occurrence when CIK cells are injected *in vivo* (see examples). CIK cells are more cytotoxic against monolayers of freshly proliferating undifferentiated EC than against contact inhibited EC monolayers that have been allowed to deposit fibronectin, laminin and collagen following extended periods of time in culture. CIK cells vigorously lyse endothelial monolayers that are either growing on deprived extracellular matrices or that are mitogenically activated. These features are common to both sub-confluent cultured EC monolayers as well as tumor angiogenesis.

Culturing EC as proliferating monolayers interferes with their differentiation and decreases fibronectin mRNA production. Under more physiologically relevant *in vitro* environments that limit cell proliferation, EC tend to organize into viable, three-dimensional tubular structures and have increased fibronectin mRNA production (Jaye et al. (1985) Science 228:882). Activation of EC with phorbol myristate acetate (PMA), a tumor promoter that markedly increases EC production of collagenase and plasminogen activator has been shown to lead to ECM degradation that precedes the invasion of perivascular spaces with sprouting neoangiogenesis, a process which can be blocked by metalloprotease inhibitor 1,10-phenanthroline (Montesano et al. (1985) Cell 42:469). The *in vitro* finding that activation of EC is linked to collagenase IV expression and synergizes with expression of mitogen basic fibroblast growth factor (bFGF) was further confirmed *in vivo* with the demonstration that bFGF and human immunodeficiency virus type 1 (HIV-1) Tat protein synergize in inducing angiogenic Kaposi's sarcoma-like lesions in mice. Synergy is due to enhanced endothelial cell growth and type-IV collagenase expression in response to bFGF mimicking extracellular matrix proteins (Ensoli et al. (1994) Nature 371:674).

Tumor Neo-Vasculature as a Selective Target of Adoptive Immunotherapy T cells

CIK cells are remarkable for their potent *in vivo* anti-tumor effect without causing measurable GvHD or VLS in murine models. This finding was first demonstrated utilizing human CIK cells in a SCID/hu *in vivo* tumor purging model for B cell lymphoma SU-DHL$_4$ (Lu et al. (1994) J. Immunol. 153:1687). In those earlier studies, the vascular walls of the treated animals were of murine origin. The absence of GvHD and VLS in that system was thus potentially attributable to a lack of effective human-to-murine cell adhesion molecule interactions. In the experiments described herein, a murine SCID system with orthotopic full-thickness human skin allografts was used to provide human cell adhesion molecules in the vessel walls of the skin grafts. The melanoma cell line WM9 is angiogenic and selectively induces outgrowth of human neo-vasculature when injected in SCID mice (Juhasz et al. (1993) Am J Pathol 143:528).

Using the murine SCID system, *ex vivo* expanded CIK cells, when injected into mice, lacked allo-reactivity, did not cause VLS, and did not damage normal vasculature of either murine or human origin. The established skin grafts did not show signs of focal CIK cell infiltration, inflammation, or GvHD. Only the human derived neo-angiogenesis of solid tumors became the selective target of CIK cell-mediated immune surveillance in the WM9-infiltrated grafts, thus demonstrating that a subset of CIK comprising EAT cells do not target vasculature that is outside the vicinity of th tumor. CIK cell targeting of physiologically normal vascular beds outside of the tumor vicinity was not detected. These results demonstrate the existence of an immune surveillance function of EAT cells, directed against tumor neo-angiogenesis. EAT cells may represent prototype *ex vivo* expanded killer cells for the adoptive anti-angiogenic therapy of cancer. This particularly sophisticated model can be used for analysis of CIK cells and other EAT cells

A Role for *Ex Vivo* Expanded EAT Cells in the Adoptive Immunotherapy of Cancer

The clinical safety of activated NK cell therapy has been a longstanding concern after reports of massive endothelial destruction and fatal VLS as clinical side effects of LAK cell and IL-2 administration (Rosenberg et al. (1987) N. Engl. J. Med. 316:889). Cumbersome cell expansion, insecurity about the availability of sufficiently high cell doses in a timely fashion, and short shelf lives of activated NK cells may also have hampered the widespread clinical acceptance of these otherwise promising cellular agents for adjuvant cancer therapy.

As described herein, the desired cytotoxic functions of direct tumor target cytotoxicity and anti-neoangiogenic recognition are not limited to activated NK/EAT cell populations. CIK EAT cultures rapidly expand to very large numbers of T cells from peripheral blood precursors and activate pathways of non-MHC class I restricted cytotoxicity against tumor and tumor neo-angiogenesis.

As described herein, at least two distinct pathways can become activated as clonal EAT cell properties. One of these pathways determines cytotoxicity against tumor targets and the other determines cytotoxicity against aberrant or neoangiogenic endothelia. EAT/CIK cells are distinct from activated NK cells in that they more selectively attack tumor vasculature but do not cause VLS or GvHD *in vivo. In vitro,* CIK cells lyse tumor targets in an ICAM-1/LFA-1 dependent fashion, and lyse EC in an ICAM-1/LFA-1 and VCAM-1/VLA-4 independent fashion. The latter was reported to be a prerequisite feature of A-NK adhesion to tumor vasculature (Melder et al. (1996) Nat. Med. 2:992). However, a correlation of A-NK adhesion to tumor vasculature has not been correlated with an improvement of cancer outcomes (Ferrara (1996) Nat. Med. 2:971).

EAT cells display high *in vivo* selectivity for tumor vasculature and mediate infiltration and destruction of neoangiogenic vessels. Previously studied subsets of activated T cells have been reported to lack the ability to adhere to endothelia (Melder et al. (1996) Nat. Med. 2:992). The target profile of *ex vivo* expanded EAT cells, their ease of generation, and their relatively minimal side effects may facilitate their clinical acceptance as anti-angiogenic agents for the immunological therapy of cancer.

Methods of Treatment

EAT cells can be administered to an individual to treat a cellular proliferative disorder. The EAT cells can be administered to the individual by a variety of routes, e.g., intramuscularly, intravenously, intraarterially, intradermally, intraperitoneally, or subcutaneously. The treatment can consist of a single administration or comprise multiple administrations given over the course of days, weeks, months, or years. The numbers of cells administered is expected to vary based upon factors such as the individual, the route of administration, and the characteristics of the disease. Dosage can be based on lytic units, e.g., by measuring activity against cultured endothelial cells and/or tumor cells (e.g., tumor cell lines).

EAT cells administered to an individual can either be autologous or allogeneic in origin. Furthermore, the EAT cell cultures can either be bulk cultures (polyclonal) or clonal EAT cell populations. In either case, the EAT cell population to be used in a given treatment can be selected based upon a particularly desirable quality. For example, EAT cells can be selected that have a particular cytotoxic potential as demonstrated in a functional assay either *in vitro* or *in vivo.* In another example, EAT cells can be selected that have an antigenic profile associated with cytotoxicity and/or the presence or absence of alloreactivity.

EAT cells can be used in monotherapy (i.e., as the only therapeutic modality) or in combination therapy with any known chemotherapy, radiation therapy, surgery, adjuvant therapy, immunological therapy, or any other clinically proven or experimental therapies. They can be administered by gel-embedding (e.g., embedded in MatriGel), encapsulation (e.g., by the calcium-alginate method). In addition, all of these methods can be used with or without the use of ultrasonication for in situ homogenization of the EAT cells and release of their cytotoxic contents.

The EAT cells can be administered intra-peritoneally, especially for ascites tumors and those with intra-peritoneal implants (such as ovarian cancer), by intra-thecal application (such as for neural cancers and/or brain metastasis), by immuno-embolization using a procedure similar to interventional radiology procedure known as chemo-embolization. In this procedure the anti-tumor agent in a high concentration is directed released into the final blood supply of the primary or metastatic tumor, then the blood vessel supporting the tumor is sealed off by means of a gel form, a coil, a clip or equivalent.

The cells can be administered by direct cannulation of tumor vasculature or a vessel supporting the tumor in conjunction with a reservoir of EAT cells. This approach allows for continuous or pulsed release of EAT cells (and other factors in the reservoir) and includes the option for a continuous or sporadic refill of the reservoir. The device can be temporary or permanent. Alternatively, EAT cells or compositions comprising them can be directly administered the tumor or to the cavity remaining after surgical excision of a tumor or parts thereof with or without the use of a catheter.

While EAT cells can simply be injected at a site remote from the tumor to be treated, they can also be surgically administered directly to the region of the tumor. Thus, a composition comprising EAT cells and matrixes, gels, solids, spheres, fibers, collagen or tissue material can be administered.

EAT cells can be used in combination with gene targeting of the cancer or of the effector cells themselves. Thus, transient or stable expression of surface markers not usually found in EAT cells can be employed. For example, CD20 can be expressed and used as a binding site for a bi-specific antibody or a multi-specific antibody.

EAT cells can be transiently or stably transfected with a suicide gene (allowing for controlled induction of cell death), Hsp47 receptor gene, a KIR gene, TCR gene, anti-idiotypic antibody gene, or a peptide display library.

EAT cells can be administered to lytic bone lesions (e.g., in breast cancer or in multiple myeloma), or into bone marrow spaces with significant disease burden of hematologic cancers suggestive of neo-vasculariiation.

In addition to *in vivo* therapeutic methods, EAT cell cultures described herein may be used to purge autologous bone marrow in the context of an autologous bone marrow transplant (BMT), e.g., for chronic myelogenous leukemia (CML) patients undergoing autologous BMT. If EAT cell clones are used in this purging process, the clones may be selected based upon particular cytotoxic profiles as determined by their cell surface markers or their potency in functional assays.

Clones may immortalized or used in an allogeneic setting as "standard donor" EAT cells for patients that are unable to donate sufficiently viable, sufficiently expandable, or sufficiently functional EAT cell precursors, e.g., when the time to treatment is relatively short (e.g., less than about 14 days). This is similar to the use of blood products that are blood group O, Rh- (universal donor).

## Methods for Assessing the Selectivity of EAT Cells

In order to identify (or test) cells for the ability to attack unwanted vasculature (e.g., tumor vasculature), the toxicity of the cells towards HUVEC cells in the presence and absence of Hsp47 can be compared. The toxicity (measured using the assay described below) in the absence of Hsp47 is a measure of the toxicity of the cells towards, tumor vasculature while the toxicity (measured using the assay described below) in the presence of Hsp47 is a measure of the toxicity of the cells towards normal vasculature.

## Examples

## Example 1: Generation and Maintenance of Cells and Cell Lines

### (A) CIK Cells

Human CIK cells were prepared from peripheral blood lymphocytes (PBL) obtained from whole venous blood and buffy coats of healthy community donors (Stanford Blood Center, Stanford, CA). PBLs were isolated using Ficoll-Hypaque (Pharmacia Fine Chemicals, Uppsala, Sweden) density gradient centrifugation. PBLs isolated according to this method were resuspended at a density of 0.5-2x10^6 cells/ml in RPMI 1640 (GIBCO-BRL/Life Technologies, Grand Island, NY) containing 50 $\mu$m $\beta$-mercapto-ethanol (ME), 100 IU penicillin-G/ml, 100 IU streptomycin/ml, and 10% fetal calf serum (FCS) (Sigma Chemical Co., St. Louis, MO). Subsequently, an enriched population of large granular lymphocytes (LGL) and T-cells was obtained by the exclusion of plastic and nylon wool adherent cells. The enriched population was cultured in a humidified incubator with 5% carbon dioxide at 37°C.

The enriched cell population was stimulated at day 0 by the addition of 1000 IU/ml recombinant human interferon gamma (rhu γ-IFN) (Genentech, San Francisco, CA). On day 1, the cell population was stimulated with: (1) 50 ng/ml of the soluble anti-human CD3 monoclonal antibody OKT-3 (derived from the hybridoma obtained from the American Tissue Culture Collection, Rockville, MD) and; (2) 300 IU/ml of recombinant human interleukin-2 (rhu IL-2) (Cetus/Chiron, Emeryville, CA). Cultures were maintained by the addition of 50% fresh medium and 300 IU rhu IL-2 every 3 to 4 days for 21 to 28 days.

On day 14, the lytic activity of CIK cells produced according to the above method was tested in $^{51}$Cr release cytotoxicity assays against tumor and EC targets.

For large scale production of CIK cells from a single donor, nylon wool non-adherent cells were cultured at 37˚C with under the CIK induction conditions described above, with the exception that cells were maintained at a density of 5-10 x$10^6$ cells/ml in turbine/impeller agitated bioreactors of 500 ml, 1,000 ml (Nalgene, Rochester, NY), and/or a larger pO$_2$ and pH controlled 15,000 ml (Chemap, Völketsvil, Switzerland) capacity.

(B) Endothelial Cells

Freshly isolated single donor human umbilical cord endothelial cells (HUVEC) or pooled donor HUVEC were (ATCC, Rockville, MD) cultured on gelatin (Sigma Chemical Co., St. Louis, MO) coated tissue culture flasks, 6-well plates, or 24-well plates (Coming, Coming, NY) in TC-199 media or Ham's F12-K media. EC culture media was supplemented with 100 IU penicillin-G/ml, 100 IU streptomycin/ml, 5 μg porcine heparin/ml, 20% FCS (Sigma Chemical Co., St. Louis, MO), and 80 μg endothelial mitogen/ml (Biomedical Technology Inc., Stoughton, MA) and cells were maintained in a humidified incubator with 5% carbon dioxide at 37˚C. Upon reaching 90% confluence, HUVEC were passaged using 0.25% trypsin and 0.02% EDTA in Ca$^{2+}$Mg$^{2+}$ free phosphate buffered saline (PBS).

(C) Tumor Targets

The hematopoietic tumor cell lines SU-DHL, OCI-Ly8, and K562 were cultured at a density of 0.5-2x$10^6$ cells/ml in RPMI 1640 medium (GIBCO-BRL/Life Technologies, Grand Island, NY) containing 50 μM β-mercapto-ethanol (ME), 100 IU penicillin-G/ml, 100 IU streptomycin/ml, and 10% FCS (Sigma Chemical Co., St. Louis, MO) in a humidified incubator with 5% carbon dioxide at 37˚C. Melanoma cell lines WM9 and 1205 LU (Dr. M. Herlyn, Wistar Institute of Anatomy and Biology, Philadelphia, PA) were maintained on tissue culture treated flasks (Beckton Dickinson, San Jose, CA) in MCDB 153 medium (Life Technologies, Grand Island, NY) supplemented with 5 μg/ml insulin and 2% FCS in a humidified incubator with 5% carbon dioxide at 37˚C.

Example 2: $^{51}$Cr-Release Cytotoxicity Assays

Target cells used in cytotoxicity assays were metabolically labeled with $^{51}$Cr (DuPont-New England Nuclear, Boston, MA) by incubating 1x$10^6$ cells with 300 mCi $^{51}$Cr at 37˚C for 1-1.5 hours. Following the incubation, the cells were washed three times with phosphate buffered saline (PBS) containing 0.1% bovine serum albumin. The labeled target cells were then distributed in triplicate in flat-bottomed 96 well microtiter plates at a concentration of 2x$10^4$ cells/ well. Effector (CIK) cells were added at the indicated ratios. Monoclonal antibodies were incubated with target cells for 15-30 minutes at room temperature prior to the addition of effector cells. The final volume of the assay mixture in each well was 0.2 ml. After 4 hours at 37˚C, the cells were collected by centrifugation and an aliquot of the supernatant was counted in a gamma counter (Micromedic Systems, Horsham, PA). The percentage of specific $^{51}$Cr release was calculated according to the following equation:

$$\% \text{ specific } ^{51}\text{Cr release} = \frac{(\text{test release}) - (\text{spontaneous release})}{(\text{maximal release}) - (\text{spontaneous release})} \times 100\%$$

Spontaneous release was determined by incubating the labeled target cells in complete medium alone. Maximal release was determined by lysing the labeled cells with 1% NP-40. Experiments were performed in triplicate assays, and bulk CIK culture data was repeated with three different random donors.

Example 3: *Ex Vivo* Expansion and Cytotoxicity Profile of CIK Cells

Three to five percent of unmanipulated peripheral blood mononuclear cells (PBMC) present a CD3$^+$56$^+$ phenotype. Preferential expansion of CD3$^+$56$^+$ cells is observable as early as day 10 of culture (using the CIK cell culture conditions of Example 1), when of 12-15% of cells stain positive for the CD3$^+$56$^+$ phenotype. Day 14 to day 28 cultures typically yielded 30-50% of CD3$^+$56$^+$ cells.

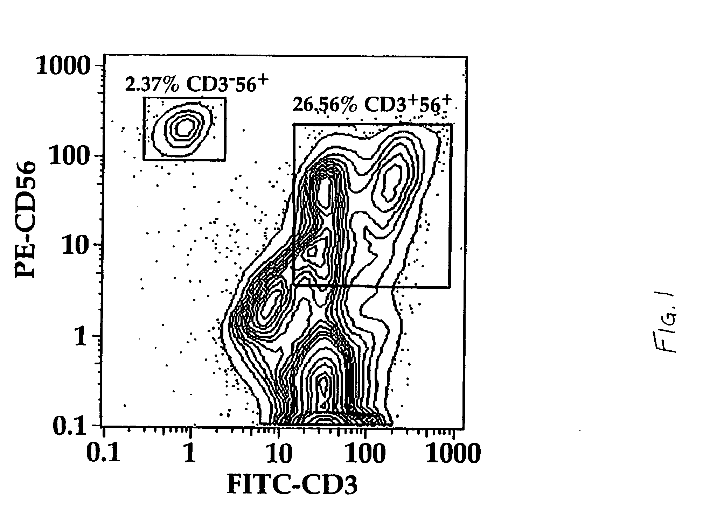

Cell surface expression of CD3 and CD56 was analyzed for mature (day 21) CIK cells that were expanded *ex vivo* in bioreactors. Approximately 10$^6$ CIK cells were stained with anti-CD56-PE and anti-CD3-FITC monoclonal antibodies according to the manufacturer's recommendations and analyzed on a FACS-Star® (Beckton-Dickinson, San Jose, CA). The contour graph of a typical batch of CIK cells (Figure 1) shows that 26.5% of the CIK cells are CD3$^+$56$^+$ and 2.3% are CD3$^-$56$^+$.

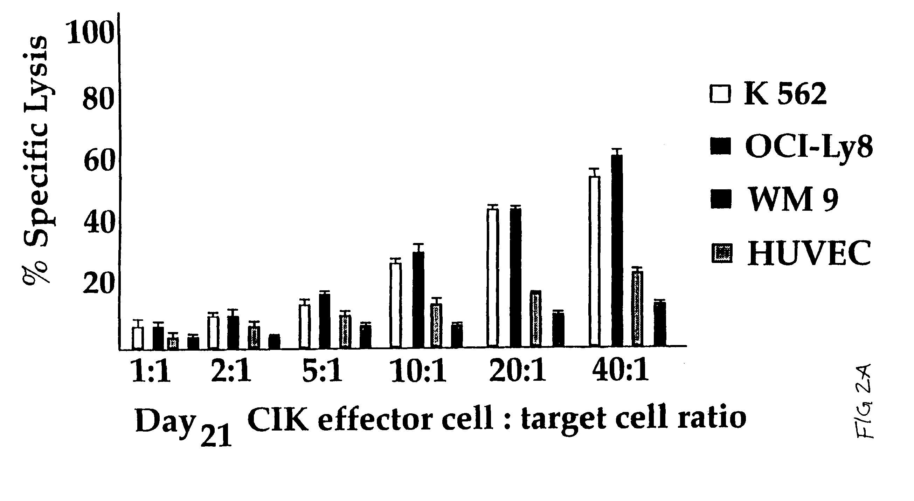

When used as effectors in $^{51}$Cr-release cytotoxicity assays, day 21 CIK cells (cultured as described in Example 1) were capable of mediating significant anti-tumor activity against the B-cell lymphoma cell lines OCI-Ly8 and SU-DHL$_4$ and against the myeloid leukemia cell line K562 (Figure 2A). In addition, CIK cells also lysed, though to a lesser extent, freshly confluent (passage number three) cultured HUVEC (Figure 2A). CIK cells displayed minimal direct cytotoxicity against the melanoma cell line WM9 (Figure 2A).

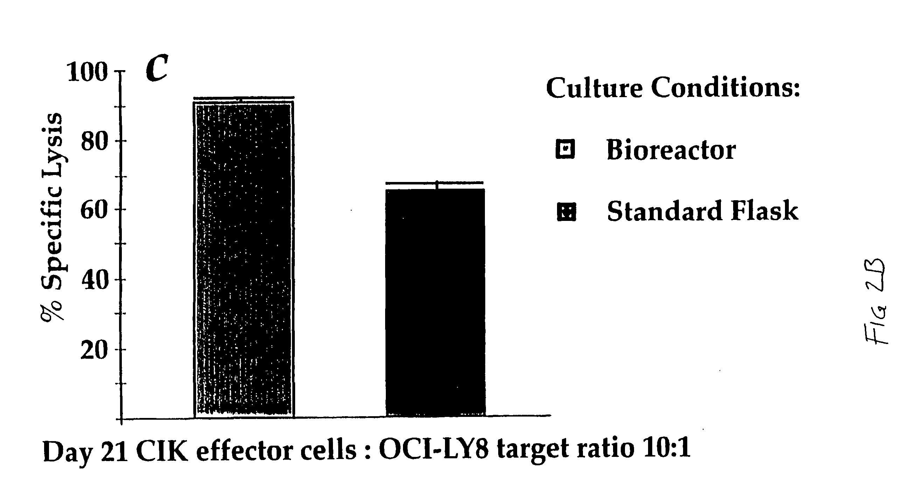

CIK cell cultures grown in impeller agitated bioreactors can be grown to a density of 5-10x 10$^6$ cells/ml, a density tenfold that of a static flask culture. The use of a 15 liter bioreactor with optional pH and pO$_2$ control allows for the expansion of up to 3x 10$^{11}$ CIK cells by day 21 from single buffy coat PBMC, with greater than 25% of the cells possessing a CD3$^+$56$^+$ phenotype. The bioreactor-grown CIK cells were compared to CIK cells cultured in conventional standing tissue culture flasks (generated from an aliquot of the same PBMC buffy coat) in a $^{51}$Cr release cytotoxicity assay against the tumor target OCI-Ly8. The lytic activity of the bioreactor-grown CIK cells exceeded that of the standard flask-grown CIK cells, as measured against the tumor target OCI-LY8 at a E:T ratio of 10:1 (Figure 2B).

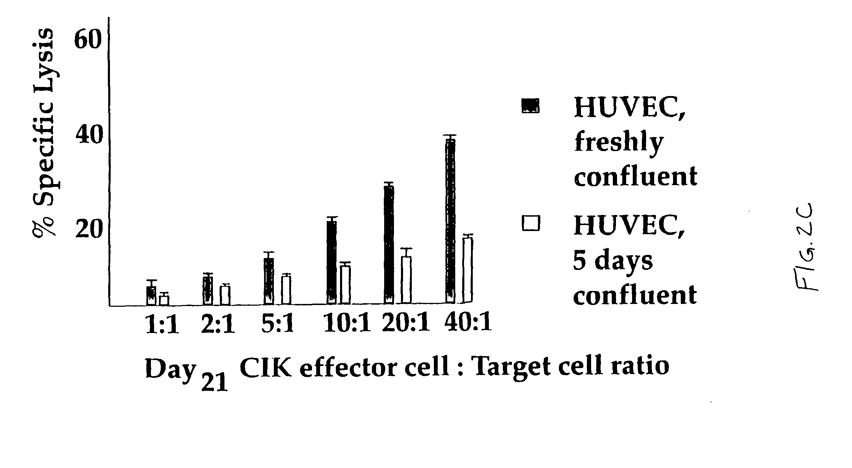

The duration of EC mololayer confluency was found to correlate with sensitivity to CIK cell cytotoxicity. Routine tryptic passaging of HUVEC was generally carried out when the cells reached more than 80% confluency. $^{51}$Cr release assays using cultured HUVEC as targets were performed at two time points: (1) when HUVEC confluency was newly reached ("freshly confluent"); and (2) when HUVEC monolayers were cultured for five days after reaching confluency (media was changed every 48 hours). Freshly confluent HUVEC were lysed by CIK cells three times more readily than HUVEC monolayers confluent for five days (Figure 2C).

Example 4: Effect of Monoclonal Antibodies on CIK Cell Mediated Lysis

A broad array of monoclonal antibodies against cell adhesion molecules (CAMs), T cell markers, and NK cell markers was tested in $^{51}$Cr release cytotoxicity assays to determine the role of these cell surface molecules in the non MHC class I restricted cytotoxicity mediated by CIK cells against tumor and EC targets. The monoclonal antibodies used in these experiments sterically hinder the interaction between receptor and ligand molecules, without stimulating receptor mediated signaling events (Shields *et al.* (1998) Tissue Antigens 51:567). Prior to the addition of effector cells to the assay, purified NaN$_3$-free monoclonal antibody was added to the target cells at a concentration of 5 $\mu$g/ml of medium and incubated at room temperature (RT) for 15 minutes. Isotype matched antibodies were used as controls. Effector cells were added directly to the treated targets at a 20:1 E:T ratio and incubated under standard for 4 hrs. The remainder of the cytotoxicity assay was performed as detailed above, at 37°C in a humidified atmosphere of 5% CO$_2$.

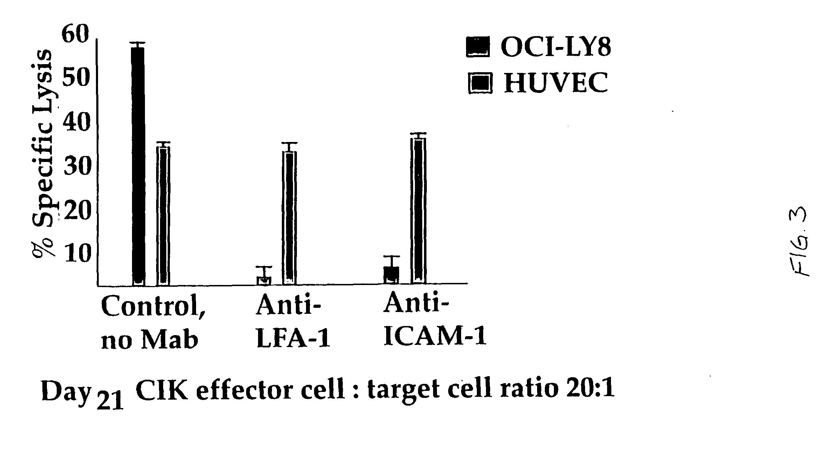

Monoclonal antibodies to MHC class I, T cell markers CD4, CD8, TcR$\alpha/\beta$ and TcR$\gamma/\delta$, and NK cell markers CD 16, CD56, NK-B1 and Nkp40 did not block cytotoxicity to either of the targets. Monoclonal antibodies to various CAMs (see Table 1) also had no effect on CIK cell-mediated cytotoxicity, with the exception of anti-ICAM-1 and anti-LFA-1 antibodies. Anti-ICAM-1 and anti-LFA-1 monoclonal antibodies significantly reduced CIK lysis of tumor targets, but not of EC targets (Figure 3 and Table 1). No monoclonal antibodies were identified in these experiments that were capable of blocking CIK cell-mediated cytotoxicity against EC targets.

| TABLE 1: CIK Cytotoxicity Blocking Studies With Monoclonal Antibodies | | | |
|---|---|---|---|
| **Molecule Class:** | **Antibody Against:** | **> 15 % Reduction Of EC Lysis** | **> 15% Reduction Of Tumor Lysis** |
| **Control** | Irrelevant Isotype | - | - |
| **MHC** | MHC I (W6/32) | - | - |

(continued)

| TABLE 1: CIK Cytotoxicity Blocking Studies With Monoclonal Antibodies | | | |
|---|---|---|---|
| **Molecule Class:** | **Antibody Against:** | **> 15 % Reduction Of EC Lysis** | **> 15% Reduction Of Tumor Lysis** |
| **T-Cell Markers** | TCR α/β | - | - |
| | TCR γ/δ | - | - |
| | CD 2 (LFA-2) | - | - |
| | CD 4 | - | - |
| | CD 8 | - | - |
| **NK-Cell Markers** | CD 16 | - | - |
| | CD 56 | - | - |
| | p40 (via MAb NKTA 255) | - | - |
| | NKB1-Kir1 (via MAb DX 9) | - | - |
| **Cell Adhesion Molecules, β2 intergrins** | ICAM-1 (CD 54) | - | + |
| | LFA-1 (CD 18/11a) | - | + |
| | CD18 (LFA-1 β-chain) | - | + |
| | CD 11a (LFA-1 α-chain) | - | + |
| | CD 11b (Mac-1 α-chain) | - | - |
| | CD 11c (CR 4 α-chain) | - | - |
| **Other CAMs** | CD 34 | - | - |
| | PECAM-1 (CD 31) | - | - |
| | VAP-1 | - | - |
| | VCAM-1 | - | - |
| | VLA-4 | - | - |
| | N-Cadherin | - | - |

Example 5: Effect of Brefeldin A and Concanamycin A on CIK Cell Mediated Lysis

Brefeldin A (BFA) blocks the transport of FasL from intracellular stores to the cell surface (Lippincott-Schwartz et al. (1989) Cell 56:801; Lippincott-Schwartz et al. (1990) Cell 60:821; and Orci et al. (1991) Cell 64:1183). BFA-treated effector cells were used in cytotoxicity assays to evaluate the fractional contribution of the Fas/FasL pathway to the total lytic activity of CIK cells against various targets. For BFA treatment of effector cells, $10^6$ CIK cells were incubated with medium supplemented with 1 μM BFA (Böhringer Mannheim GmbH, Mannheim, Germany) and incubated for 2 hours in a humidified incubator with 5% carbon dioxide at 37˚C. BFA was dissolved at 100 μg/ml in 70% ethanol and used fresh or stored up to 4 weeks at -20˚C. Prior to cytotoxicity assays, the cells were rinsed three times with PBS and BFA-free fresh medium was added.

Concanamycin A (conA) depletes perforin contained in cytotoxic T-cell granules (Kataoka et al. (1996) J. Immunol. 156:3678; Ohminami et al. (1999) Blood 93:925). ConA-treated effector cells were used in cytotoxicity assays to approximate the fractional contribution of the perforin/granzyme pathway to the total CIK cell mediated cytotoxicity. For conA treatment of effector cells, CIK cells were washed and resuspended at $1 \times 10^6$ cells/ml in complete medium containing 0.1 μM conA (Sigma, St. Louis, MO). The cells were then incubated for 2 hours at 37˚C in a humidified atmosphere of 5% $CO_2$. At the end of the incubation, the CIK cells were washed and used as effectors in cytotoxicity assays.

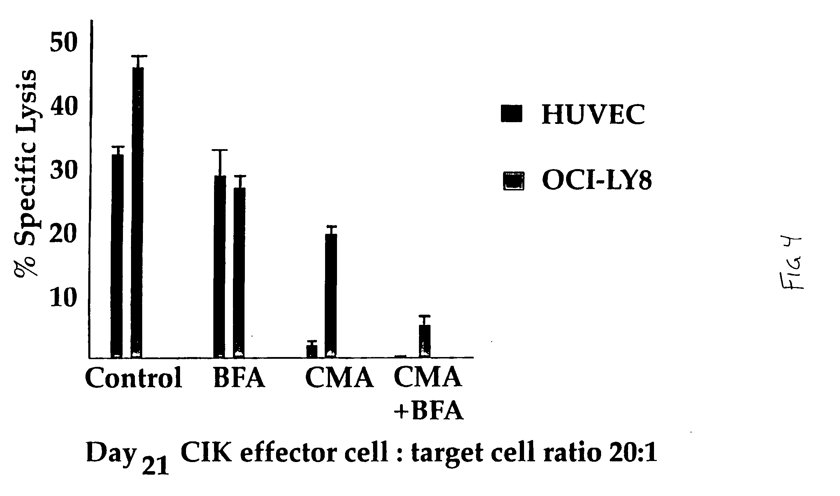

Day 21 CIK cells were pre-incubated with BFA, conA, or both, then washed and assayed against tumor cell and EC targets. BFA treatment of effector cells and the associated block of FasL transport reduced CIK lysis of the tumor target OCI-Ly8 by 33%, but had no effect on the lysis of EC targets (Figure 4). Con A treatment of effector cells and the associated depletion of CIK perforin stores reduced OCI-Ly8 tumor lysis by 66% and completely abolished EC lysis (Figure 4).

Example 6: Clonal Mechanisms for EC and Tumor Target Cytotoxicity

CIK cell clones were generated as follows. CD3$^+$CD4$^-$8$^-$ cells were obtained by FACS sorting of the nylon wool non-adherent cell population described above in Example 1. For FACS analysis and sorting, approximately 10$^6$ cells were washed twice with Ca$^{2+}$, Mg$^{2+}$, and 5% FCS-containing PBS buffer, suspended in 50 $\mu$l of the buffer, and placed at 0-4˚C in the dark. 1 $\mu$g of FITC, PE-, Per-CP or TriColor labeled monoclonal antibody (Beckton Dickinson, San Jose, CA) per staining reaction of 10$^6$ cells was mixed with the cells and incubated for 15 to 30 minutes at 0-4˚C in the dark. Stained cells were subjected to three subsequent washes with 10 volumes of Ca$^{2+}$, Mg$^{2+}$, and 5% FCS containing PBS and taken up in 500 $\mu$l of this buffer for analysis or sort by FACScan/FACStar (Beckton Dickinson, San Jose, CA) with excitation at 488 nm and the appropriate channel filters for detection of emission.

Bulk cultures of the CD3$^+$CD4$^-$8$^-$ sorted cells were maintained for 72 hours under the CIK induction conditions described above. Following this culture period, CIK clones were obtained by limiting dilution consisting of depositing a mean of 0.6 cells per well of a 96 well plate. The wells contained twice-irradiated CIK feeder cell cultures at 1x 10$^6$ cells/ml and were supplied with fresh IL-2 at 300 IU/ml every five days of culture.

Clonal populations of CIK cells were expanded to more than 2x 10$^6$ cells per culture. When used as effectors in cytotoxicity assays, target specificity varied with individual clones. The lytic specificities of CIK clones were as follows: (1) lysis of tumor targets but not EC targets; (2) lysis of EC targets but not tumor targets; or (3) lysis of both tumor targets and EC targets.

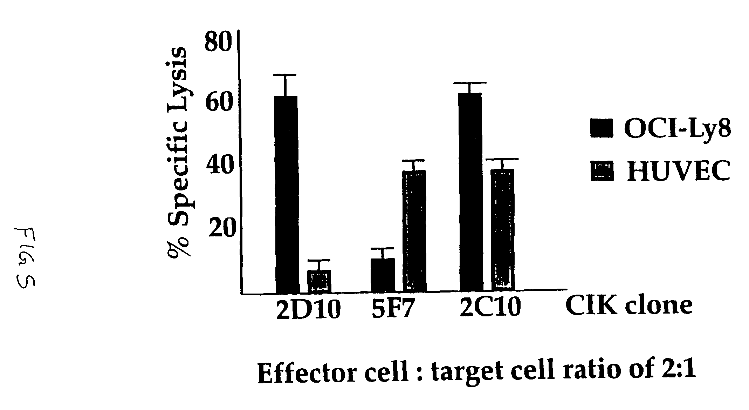

The lytic properties of three representative clones are shown in Figure 5. Clone 2D10 recognized OCI-Ly8 with >60% specific lysis at an E:T ratio of 2:1 and did not recognize HUVEC. Clone 5F7 exhibited potent cytotoxicity against cultured HUVEC and minimal activity against OCI-Ly8. Clone 2C10 recognized both tumor and endothelial targets efficiently. The dual specificity of clone 2C10 was the most common type observed.

Example 7: CIK Cell-Mediated Adoptive Immunotherapy

The ability of CIK cells to selectively destroy tumor cells *in vivo* was tested in a mouse model comprising orthotopically grown human full-thickness skin grafts, orthotopically grown human nodular melanoma WM9, and intra-peritoneally injected CIK cells. This model was established to evaluate under physiologic conditions CIK cell-mediated direct anti-solid tumor activity as well as anti-neo-angiogenic activity directed against tumor vasculature. The human-derived melanoma cell line WM9 is angiogenic when propagated in mice, selectively inducing the outgrowth of human, but not murine derived, neo-vasculature (Juhasz et al. (1993) Am J Pathol 143:528).

The mouse model system was established as follows. Six week old female SCID mice (Dr. I. Weissman, Stanford University, Stanford, CA) were irradiated at 500 Rads one day prior to skin grafting. Human full thickness foreskin grafts were prepared by aseptically exposing their subcutaneous vascular beds, and trimming their edges to yield a 20 x 30 mm graft. Foreskin samples were rejected that yielded smaller grafts or that had a shelf age of greater than 24 hours. Anesthetized animals were prepared in a 37˚C heated operative environment by aseptically denuding matching areas on their flanks. The full-thickness skin grafts were placed with interrupted absorbable suture, aseptically bandaged, and the grafted animals were housed separately until full engraftment was evident.

After engraftment, 10$^5$ WM9 melanoma cells suspended in PBS were injected intradermally into a single site of the human full-thickness skin of triplicate animals. Tumor growth was monitored by observation and palpation. Animals with evident nodular tumor burden exceeding a diameter of 5 mm were injected intraperitoneally with 10$^7$ CIK cells (suspended in RPMI without FCS or IL-2) of proven lytic activity in same day $^{51}$Cr release cytotoxicity assay at CIK culture day 14-21. Animals were sacrificed at 0, 24, 48, and 72 hours following CIK injection. Internal organs and the human skin/melanoma graft were fixed and analyzed by light microscopy of H&E and immuno-peroxidase anti-human CD3, human PE-CAM and human ker antigens stained tissue sections (Dr. O. Kamel, Stanford University, Stanford, CA).

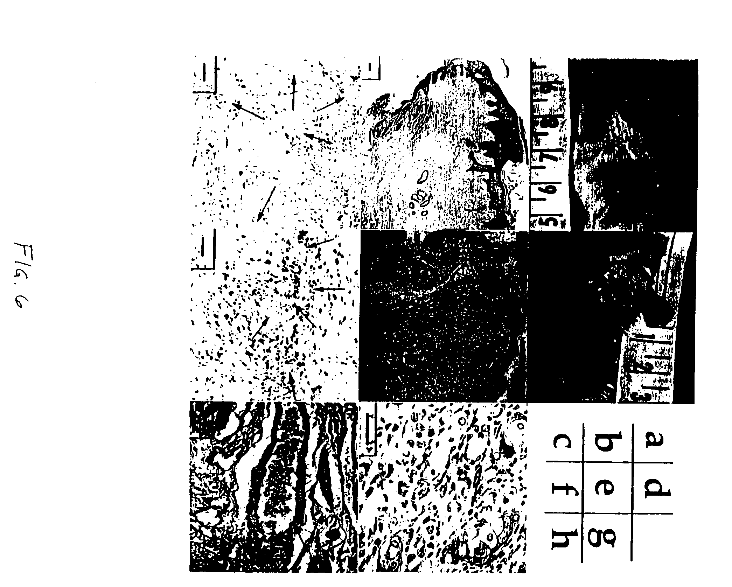

Within six weeks of the initial skin graft, full thickness human skin grafts were established on the dorsum of the host SCID mice (Figure 6a) and displayed normal human histology (Figure 6b). When injected intradermally with human nodular melanoma WM9 cells, numerous nodular melanoma solid tumor nests developed in the cutis of the full thickness grafts (Figure 6d, 6e). The human origin of the outgrowing tumor neo-vasculature was confirmed by histologic immunodetection (IPOX) using species specific anti-human PECAM-1 monoclonal antibody (Figure 6h). Histo-micrographs demonstrated that these nodular tumor masses invaded the surrounding outgrowing human derived capillaries (Figure 6g). No metastasis to murine tissues was observed.

In the above experiments, approximately 10$^7$ mature CIK cells were injected into the peritoneal cavity of mice bearing either: (1) established human full thickness skin grafts; or (2) human full thickness skin grafts with orthotopic nodular melanoma burden. Skin grafts with and without tumor burden were harvested at day 3 after intraperitoneal injection of CIK cells. None of the mice developed signs of vascular leak syndrome. There was no visible evidence of alloreactive skin infiltration by CIK cells or accompanying inflammation at any time point after CIK cell injection in either the control or melanoma skin grafts (Figure 6c, 6f). The injected human CIK cells were detected by anti-human CD3 immunohistochemistry.

CIK cells selectively migrated to vascular beds supplying the tumor nodules (Figure 6f). The perimeter of the tumor vasculature became surrounded by a concentric dense cellular infiltrate of human CD3$^+$ staining lymphocytes. CD3$^+$ cells selectively transmigrated to the tumor vicinity and caused edema and neo-vascular tissue destruction. The vasculature and perivascular spaces of the control skin grafts showed only minimal background CD3$^+$ staining (Figure 6c). The numerous endothelial linings of the control graft's dermal vessels remained intact without focal lymphocytic infiltrates. The murine atrophic spleen, lung and bone marrow similarly lacked lymphocytic infiltrates. CIK cells co-localized with melanoma cells invading the endothelialized capillaries, however direct tumor lysis was not evident (Figure 6g).

Example 8: Brefeldin A Protects EC from Non-MHC I Restricted Killing

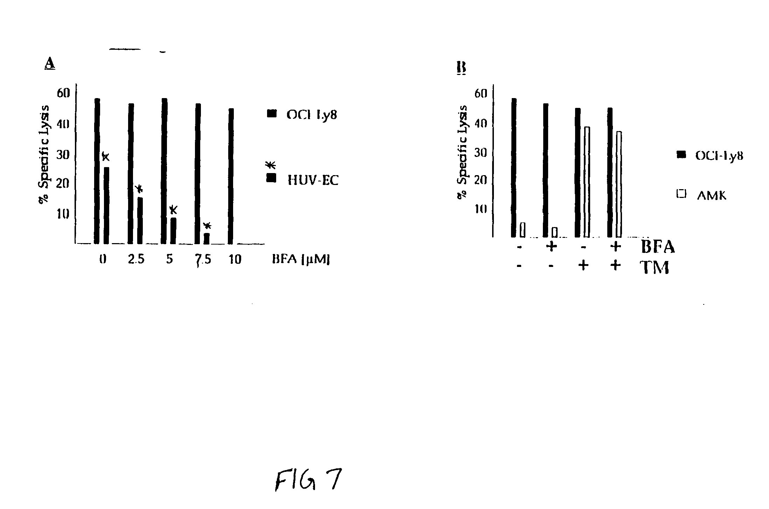

Figure 7 illustrates the results of an experiment demonstrating that brefeldin A (BFA; a compound which blocks transport of proteins from the golgi to the ER) protects EC from non-MHC I restricted killing. CIK targets were treated with BFA and/or tunicamycin (TM). Pre-treatment with BFA did not reduce killing of tumor or lymphoblastoid targets. However, it did suppress CIK mediated lysis of EC in a $^{51}$Cr release assay (20:1 E:T ratio) with complete protection at 10 $\mu$M BFA. This resistance is sustained after BFA is removed from the media. Resistant lymphoblastoid target AMK were rendered susceptible by a 22 hr TM pre-treatment that was not reversed by BFA treatment. Neither TM or TM and BFA together altered CIK mediated lysis of OCI-Ly8 tumor targets.

Treatment of EC with BFA increases extracellular levels of Hsp47, leading to the protective effect. Additional protective molecules can be identified by treating cells with BFA and then screening for protective agents in culture or extract of BFA treated EC.

Example 9: A Bacterially Expressed rhuHsp47-GST Fusion Protein Protects EC from Lysis by CIK in a Dose-Dependent Manner

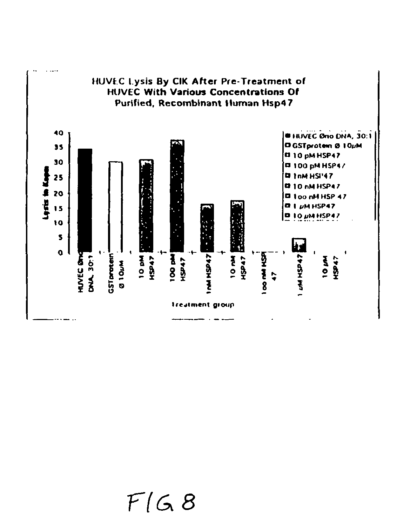

A $^{51}$Cr release assay (20:1 E:T ratio) demonstrated that affinity purified rhuHsp47-GST fusion protein protects EC from CIK mediated lysis (Figure 8). Control GST protein had no effect on lysis. However, as little as 1 nM rhuHsp47-GST fusion protein reduced EC cell lysis by 50%, and pre-incubation with 100 $\mu$M rhuHsp47-GST fusion protein completely protected EC from CIK mediated lysis. In this study, the cells were washed after incubation with fusion protein and prior to exposure to CIK, demonstrating Hsp47 interacts with EC or extracellular matrix is effective during the incubation period.

Example 10: A GFP-tagged rhuHsp47-GST Fusion Protein Identifies a 26% Subset of CIK Cells

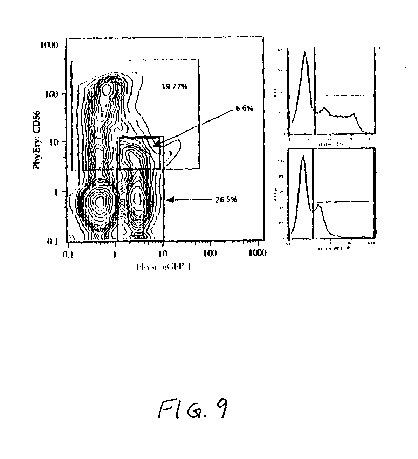

As shown in Figure 9, FACS analysis CIK cells (21 day culture) with a GFP-tagged rhuHsp47-GST fusion protein and CD56-PE revealed that 26% of CIK cells stained with the Hsp47 fusion protein, 39.7% stained with the CD56 fusion protein and 6.6% stained with both.

Example 11: EC Cells Transfected with Hsp47, but not with a RDEL Deletion of Hsp47 are Protected from CIK Mediated Lysis

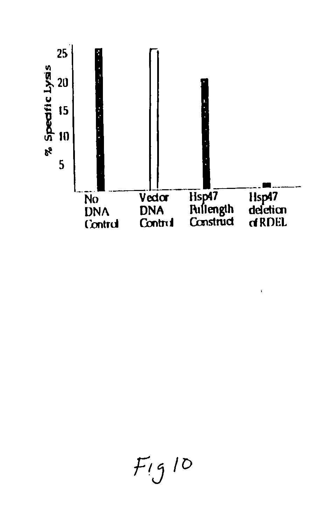

EC were transfected either with full-length Hsp47 or a Hsp47 mutant lacking the C-terminal ER retention sequence RDEL. The cells were exposed to CIK cells and a standard $^{51}$Cr release assay (20:1 E:T ratio) was performed. As shown in Figure 10, about 25% of control EC cells were lysed, about 20% of EC expressing full-length Hsp47 were lysed, and virtually none of the EC expressing the mutant Hsp47 were lysed. This increase in protection arises from the fact that truncation of the RDEL directs Hsp47 production to secretion. This is the first demonstration that otherwise universally sensitive EC can be rendered resistant to lysis by non-MHC restricted killer cells via expression of a transgene.

Example 11: An HLA-A Consensus Peptide Protects EC from CIK Mediated Lysis in a Dose Responsive Manner and Equivalently to Hsp47

Hsp47 has some homology to HLA-A2 suggesting that a consensus peptide could protect EC from CIK mediated lysis. A peptide (AVLSAEQRL) having the sequence of a region of Hsp47 that is homologous to a portion of HLA-A2 was generated. EC were exposed to CIK cells in the presence of the consensus peptide or an irrelevant control peptide and a standard $^{51}$Cr release assay (20:1 E:T ratio) was performed. The consensus peptide protected EC in a dose-dependent manner.

Other Embodiments

It is to be understood that while the invention has been described in conjunction with the detailed description thereof, that the foregoing description is intended to illustrate and not limit the scope of the appended claims. Other aspects, advantages, and modifications are within the scope of the following claims.

**Claims**

1.   A method for preparing an immune cell composition for the treatment of vascularized cancer, that comprises immune cells that recognize Hsp47 or a peptide with the sequence AVLSAEQRL as a non-kill signal and thereby have the ability to selectively damage tumor vasculature and not normal vasculature with

   a. the step of ex vivo expanding the immune cell composition generated from a hematopoietic cell sample in a closed culture system comprising IL2, OKT3 and gamma interferon;
   b. the step of assessing the selectivity of the expanded cells with respect to the lytic activity against tumor vasculature as compared to the lytic activity against normal vasculature
   c. the step of adjusting the selectivity of the composition.

2.   An immune cell composition for the treatment of vascularized cancer that comprises CIK cells that recognize Wsp47 or a peptide with the sequence AVLSAEQRL as a non kill signal and thereby have the ability to selectively damage tumor vasculature and not normal vasculature wherein the lytic activity of the composition is adjusted so that at least 50% of the ex vivo expanded cells selectively kill tumor-associated vascular endothelial cells compared to vascular endothelial cells associated with normal tissues.

3.   An immune cell composition produced by the method of claim 2 wherein the ex vivo expanded cells comprise cells expressing both CD3 and CD56.

4.   An immune cell composition produced by the method of claim 2 wherein the ex vivo expanded cells comprise cells that kill tumor cells.

5.   An immune cell composition produced by the method of claim 2 further comprising a chemotherapeutic compound.

6.   An immune cell composition produced by the method of claim 2 further comprising an agent which links the ex vivo expanded cells to a selected compound.

7.   The immune cell composition of claim 6 wherein the agent is a bi-specific antibody.

8.   The immune cell composition of claim 6 wherein the agent comprises at least two covalently bound antibodies.

9.   The immune cell composition of claim 6 wherein the agent comprises an antibody which binds to the ex vivo expanded cells.

10.   The immune cell composition of claim 9 wherein the selected compound is covalently bound to the antibody.

11.   The immune cell composition of claim 9 wherein the selected compound is non-covalently bound to the antibody.

12.   The immune cell composition of claim 6 wherein the selected compound is selected from the group consisting of a toxin, an antibody, a detectably labelled molecule, an immuno-modulator, and a radioactive compound.

13.   Use of a composition of any one of claims 2 to 12 for the preparation of a pharmaceutical composition for treating cancer that stimulates neo-angiogenesis, wherein the ex vivo expanded cells are autologous to the patient.

14.   The use of claim 13 wherein the tumor is a solid tumor.

15.   The use of claim 13 wherein the ex vivo cells are capable of undergoing replication in culture.

**16.** use of claim 13 wherein the composition is administered without co-administration of a cytokine.

**17.** The use of claim 13 wherein the composition is not administered within five days of the administration of a cytokine.

**18.** The use of claim 13 wherein at least $10^5$ of the ex vivo expanded cells are administered in a given day.

**19.** The use of claim 13 where the composition is administered at least twice within 7 days.

**20.** The use of claim 13 where the composition is administered at least twice within 30 days.

**21.** The use of claim 13 wherein the patient is suffering from a cancer selected from a stage 1 cancer, stage 2 cancer, stage 3 cancer, or a stage 4 cancer.

**22.** The use of claim 13 wherein the patient is suffering from a cancer selected from a low grade cancer, an intermediate grade cancer, and a high grade cancer.

**23.** The method of claim 1 wherein the cells are grown in a membrane enclosure.

**24.** The method of claim 1 for ex vivo expansion of immune cells with an Hsp47 receptor comprising culturing the cells in a closed system bioreactor.

**25.** The use of claim 13 wherein treatment comprises outpatient treatment.

**26.** The use of claim 13 wherein the patient is a cancer survivor.

**27.** The use of claim 13 wherein the patient is healthy.

**28.** The use of claim 13 wherein the patient is at increased risk for cancer.

**29.** A use of a composition with a defined selective lytic activity of any of claims 2-12 for preparation of a pharmaceutical composition for treating a vascularized cancer wherein the ex vivo expanded cells are allogenic to the patient.

**30.** The use of claim 29 wherein the cells are immortalized.

**31.** The composition of any of the claims 2-12 wherein the ex vivo expanded cells harbour a stable transgene comprising a regulable suicide gene.

**Patentansprüche**

**1.** Eine Methode zur Herstellung einer Immunzellzusammensetzung zur Behandlung von vaskularisiertem Krebs, die Immunzellen umfasst die Hsp47 oder ein Peptid mit der Sequenz AVLSAEQLR als "Nicht-Kill Signal" erkennen und somit die Fähigkeit haben, selektiv Tumorvaskulatur zu schädigen, aber nicht normale Vaskulatur, mit

a. dem Schritt einer ex vivo Vermehrung von Immunzell-Zusammensetzungen, die aus einem hämatopoietischem Zellmaterial generiert sind in einem geschlossenen Zellkultursystem mit Interleukin 2 (IL-2), monoklonalem Antikörper OKT-3, und Gamma-Interferon;
b. dem Schritt, die Selektivität der expandierten Zellen zu bestimmen bezüglich ihrer lytischen Aktivität gegen Krebsblutgefäße im Vergleich zu ihrer lytischen Aktivität gegen normale Blutgefäße;
c. dem Schritt der Anpassung der selektiven Aktivität der Zusammensetzung.

**2.** Eine Immunzellzusammensetzung zur Behandlung von vaskularisiertem Krebs, die CIK-Zellen umfasst die Hsp47 oder ein Peptid mit der Sequenz AVLSAEQLR als "Nicht-Kill Signal" erkennen und somit die Fähigkeit haben, selektiv Tumorvaskulatur zu schädigen, aber nicht normale Vaskulatur, wobei die lytische Aktivität der Zusammensetzung so angepasst ist, dass mindestens 50% der ex vivo vermehrten Zellen selektiv vaskuläre tumorbezogene vaskuläre Endothelzellen zerstören, im Vergleich zu vaskulären Endothelzellen, die in normalen Geweben vorkommen.

EP 1 231 840 B1

3. Eine Immunzellzusammensetzung hergestellt nach Anspruch 2, wobei die ex vivo vermehrte Zellpopulation Zellen umfasst, die sowohl CD3 als auch CD56 exprimieren.

4. Eine Immunzellzusammensetzung hergestellt nach Anspruch 2, wobei die ex vivo vermehrten Zellen Zellen umfassen, die Tumorzellen zerstören.

5. Eine Immunzellzusammensetzung hergestellt nach Anspruch 2, die zusätzlich einen chemotherapeutischen Stoff umfasst.

6. Eine Immunzellzusammensetzung hergestellt nach Anspruch 2, die zusätzlich eine Substanz umfasst, die die ex vivo vermehrten Zellen mit einem ausgewählten Stoff als Linkersubstanz verbindet.

7. Die Immunzellzusammensetzung nach Anspruch 6, wobei die Substanz ein bispezifischer Antikörper ist.

8. Eine Immunzellzusammensetzung nach Anspruch 6, wobei die Substanz mindestens zwei kovalent gebundene Antikörper umfasst.

9. Eine Immunzellzusammensetzung nach Anspruch 6, wobei die Substanz einen Antikörper umfasst, der an die ex vivo expandierten Zellen bindet.

10. Eine Immunzellzusammensetzung nach Anspruch 9, wobei der ausgewählte Stoff kovalent an den Antikörper gebunden ist.

11. Eine Immunzellzusammensetzung nach Anspruch 9, wobei der ausgewählte Stoff nicht kovalent an den Antikörper gebunden ist.

12. Eine Immunzellzusammensetzung nach Anspruch 6, wobei der ausgewählte Stoff ausgewählt ist aus der Stoffgruppe bestehend aus einem Toxin, einem Antikörper, einem mit einer nachweisbaren Substanz markierten Molekül, und einen Immunmodulator, und einer radioaktiven Substanz.

13. Verwendung einer Zusammensetzung nach einem der Ansprüche von 2-12 für die Herstellung einer pharmazeutischen Zusammensetzung zur Behandlung von Krebs, der die Bildung von Neo-Angiogenese stimuliert, wobei die ex vivo vermehrten Zellen autolog in Bezug auf den Patienten sind.

14. Verwendung nach Anspruch 13, wobei der Tumor ein Festtumor ist.

15. Verwendung nach Anspruch 13, wobei die ex vivo Zellen in der Lage sind, sich während der Zellkultur zu teilen.

16. Verwendung nach Anspruch 13, wobei die Zusammensetzung ohne gleichzeitige Gabe von eines Cytokinins verabreicht wird.

17. Verwendung nach Anspruch 13, wobei die Zusammensetzung nicht innerhalb von 5 Tagen nach einer Cytokiningabe verabreicht wird.

18. Verwendung nach Anspruch 13, wobei mindestens $10^5$ der ex vivo vermehrten Zellen pro Therapietag verabreicht wird.

19. Verwendung nach Anspruch 13, wobei die Zusammensetzung mindestens zweimal innerhalb von 7 Tagen verabreicht wird.

20. Verwendung nach Anspruch 13, wobei die Zusammensetzung mindestens zweimal innerhalb von 30 Tagen verabreicht wird.

21. Verwendung nach Anspruch 13, wobei der Patient ein Krebsleiden hat, das sich entweder im Stadium ("oncologic stage") 1, Stadium 2, Stadium 3, oder im Stadium 4 des Krebsverlaufs befindet.

22. Verwendung nach Anspruch 13, wobei der Patient ein Krebsleiden hat, ausgewählt aus einem Krebsleiden niedrigen Grades, intermediären Grades, oder hohen Grades.

**23.** Die Methode nach Anspruch 1, wobei die Zellen von einem Membranbehältnis umschlossen wachsen.

**24.** Die Methode nach Anspruch 1 für die ex vivo Vermehrung von Immunzellen mit einem Hsp47-Rezeptor die umfasst, dass die Zellen in einem auf einem "closed system" Bioreaktor kultiviert werden.

**25.** Die Verwendung nach Anspruch 13, die eine "out patient" Behandlung umfasst.

**26.** Die Verwendung nach Anspruch 13, wobei der Patient Krebs überlebt hat.

**27.** Die Verwendung nach Anspruch 13, wobei der Patient gesund ist.

**28.** Die Verwendung nach Anspruch 13, wobei der Patient ein erhöhtes Krebsrisiko hat.

**29.** Verwendung einer Zusammensetzung mit einer definierten selektiven lytischen Aktivität nach Ansprüchen 2-12 zur Herstellung einer pharmazeutischen Zusammensetzung zur Behandlung von vaskularisiertem Krebs, wobei die ex vivo vermehrten Zellen für den Patienten allogen sind.

**30.** Die Verwendung nach Anspruch 29, wobei die Zellen immortalisiert sind.

**31.** Die Verwendung nach Ansprüchen 2-12, wobei die ex vivo expandierten Zellen ein stabiles Transgen mit einem regulierbaren Suizidgen aufweise.


**Revendications**

**1.** Procédé de préparation d'une composition de cellules immunes destinée au traitement d'un cancer vascularisé, qui comprend des cellules immunes qui reconnaissent l'Hsp47 ou un peptide comportant la séquence AVLSAEGRL en tant que signal d'interdiction de tuer et en conséquence ont la capacité d'endommager sélectivement le système vasculaire de la tumeur et non pas le système vasculaire normal comportant

   a. l'étape consistant à développer ex vivo la composition de cellules immunes générée à partir d'un échantillon de cellules hématopoïétiques dans un système de culture fermé comprenant de l'IL2, de l'OKT3 et de l'interféron gamma,
   b. l'étape consistant à évaluer la sélectivité des cellules développées ou qui se sont étendues par rapport à l'activité de lyse contre le système vasculaire de la tumeur telle qu'elle est comparée à l'activité de lyse contre le système vasculaire normal,
   c. l'étape consistant à ajuster la sélectivité de la composition.

**2.** Composition de cellules immunes destinée au traitement d'un cancer vascularisé qui comprend des cellules CIK qui reconnaissent le Hsp47 ou un peptide comportant la séquence AVLSAEQRL en tant que signal d'interdiction de tuer et en conséquence ont la capacité d'endommager sélectivement le système vasculaire de la tumeur et non pas le système vasculaire normal, où l'activité de lyse de la composition est ajustée de telle sorte qu'au moins 50 % des cellules développées ou qui se sont étendues ex vivo tuent sélectivement les cellules endothéliales vasculaires associées à un cancer par comparaison aux cellules endothéliales vasculaires associées aux tissus normaux.

**3.** Composition de cellules immunes produite par le procédé selon la revendication 2, dans laquelle les cellules développées ou qui se sont étendues ex vivo comprennent des cellules exprimant à la fois CD3 et CD56.

**4.** Composition de cellules immunes produite par le procédé selon la revendication 2, dans laquelle les cellules développées ou qui se sont étendues ex vivo comprennent des cellules qui tuent les cellules tumorales.

**5.** Composition de cellules immunes produite par le procédé selon la revendication 2, comprenant en outre un composé chimiothérapeutique.

**6.** Composition de cellules immunes produite par le procédé selon la revendication 2, comprenant en outre un agent qui relie les cellules développées ou qui se sont étendues ex vivo à un composé sélectionné.

**7.** Composition de cellules immunes selon la revendication 6, dans laquelle l'agent est un anticorps bispécifique.

**8.** Composition de cellules immunes selon la revendication 6, dans laquelle l'agent comprend au moins deux anticorps liés de manière covalente.

**9.** Composition de cellules immunes selon la revendication 6, dans laquelle l'agent comprend un anticorps qui se lie aux cellules développées ou qui se sont étendues ex vivo.

**10.** Composition de cellules immunes selon la revendication 9, dans laquelle le composé sélectionné est lié de manière covalente à l'anticorps.

**11.** Composition de cellules immunes selon la revendication 9, dans laquelle le composé sélectionné est lié de manière non covalente à l'anticorps.

**12.** Composition de cellules immunes selon la revendication 6, dans laquelle le composé sélectionné est sélectionné à partir du groupe constitué d'une toxine, d'un anticorps, d'une molécule marquée de manière détectable, d'un immuno-modulateur et d'un composé radioactif.

**13.** Utilisation d'une composition selon l'une quelconque des revendications 2 à 12 pour la préparation d'une composition pharmaceutique destinée au traitement du cancer qui stimule la néo-angiogénèse, où les cellules développées ou qui se sont étendues ex vivo sont autologues vis-à-vis du patient.

**14.** Utilisation selon la revendication 13, où la tumeur est une tumeur solide.

**15.** Utilisation selon la revendication 13, dans laquelle les cellules ex vivo sont capables de subir une réplication ou multiplication en culture.

**16.** Utilisation selon la revendication 13, dans laquelle la composition est administrée sans administration conjointe d'une cytokine.

**17.** Utilisation selon la revendication 13, dans laquelle la composition n'est pas administrée à moins de cinq jours de l'administration d'une cytokine.

**18.** Utilisation selon la revendication 13, dans laquelle au moins $10^5$ des cellules développées ou qui se sont étendues ex vivo sont administrées en un jour donné.

**19.** Utilisation selon la revendication 13, dans laquelle la composition est administrée au moins deux fois en l'espace de 7 jours.

**20.** Utilisation selon la revendication 13, où la composition est administrée au moins deux fois en l'espace de 30 jours.

**21.** Utilisation selon la revendication 13, où le patient souffre d'un cancer sélectionné parmi un cancer de stade 1, un cancer de stade 2, un cancer de stade 3 ou un cancer de stade 4.

**22.** Utilisation selon la revendication 13, dans laquelle le patient souffre d'un cancer sélectionné à partir d'un cancer de faible niveau ou classe, d'un cancer de niveau ou classe intermédiaire et d'un cancer de niveau ou classe élevé.

**23.** Procédé selon la revendication 1, dans lequel les cellules sont mises à croître dans une enceinte à membrane.

**24.** Procédé selon la revendication 1 pour le développement ou l'expansion ex vivo de cellules immunes comportant un récepteur d'Hsp47 comprenant la mise en culture des cellules dans un bioréacteur à système fermé.

**25.** Utilisation selon la revendication 13, où le traitement comprend le traitement d'un malade de consultation externe.

**26.** Utilisation selon la revendication 13, où le patient a survécu à un cancer.

**27.** Utilisation selon la revendication 13, où le patient est en bonne santé.

**28.** Utilisation selon la revendication 13, où le patient présente un risque accru de cancer.

**29.** Utilisation d'une composition présentant une activité de lyse sélective définie selon l'une quelconque des revendications 2 à 12 en vue de la préparation d'une composition pharmaceutique destinée à traiter un cancer vascularisé, où les cellules développées ex vivo présentent un caractère allogène vis-à-vis du patient.

**30.** Utilisation selon la revendication 29, où les cellules sont rendues immortelles.

**31.** Composition selon l'une quelconque des revendications 2 à 12, dans laquelle les cellules développées ou qui se sont étendues ex vivo abritent un transgène stable comprenant un gène de suicide pouvant être régulé.

2.37% CD3⁻56⁺

26.56% CD3⁺56⁺

F $I_G$. $I$

FIG 2A

**Day 21 CIK effector cells : OCI-LY8 target ratio 10:1**

FIG 2B

FIG. 2C

Day 21 CIK effector cell : target cell ratio 20:1

FIG. 3

EP 1 231 840 B1

FIG 4

FIG 5

FIG. 6

FIG 7

HUVEC Lysis By CIK After Pre-Treatment of
HUVEC With Various Concentrations Of
Purified, Recombinant Human Hsp47

FIG 8

FIG. 9

Fig 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Sullivan et al.** *Blood,* 1989, vol. 73, 1720
- **Weiden et al.** *N. Engl. J. Med,* 1979, vol. 300, 1068
- **Antin.** *Blood,* 1993, vol. 82, 2273
- **Hauch et al.** *Blood,* 1990, vol. 75, 2250
- **Martin et al.** *Blood,* 1985, vol. 66, 664
- **Kolb et al.** *Blood,* 1995, vol. 86, 2041
- **Porter et al.** *N. Engl. J. Med.,* 1994, vol. 330, 100
- **Rosenberg et al.** *J. Exp. Med.,* 1985, vol. 161, 1169
- **Siegel et al.** *J. Clin. Oncol.,* 1991, vol. 9, 694
- **Kotasek et al.** *Cancer Res,* 1988, vol. 48, 5528
- **Rosenberg et al.** *N. Engl. J. Med.,* 1988, vol. 319, 1676
- **Lu et al.** *J.Immunol.,* 1994, vol. 153, 1687
- **Molema et al.** *J. Controlled Release,* 2000, vol. 64, 229-39
- **Schmidt-Wolf et al.** *Br. J. Cancer,* 1999, vol. 81, 1009
- **Hoyle et al.** *Blood,* 1998, vol. 92, 3318
- **Scheffold et al.** *Bone Marrow Transplant.,* 1995, vol. 15, 33
- **Madrenas et al.** *Proc. Natl. Acad. Sci. U S A,* 1996, vol. 93, 9736
- **Wulfing et al.** *Science,* 1998, vol. 282, 2266
- **Wulfing et al.** *Proc. Natl. Acad. Sci. U S A,* 1998, vol. 95, 6302
- **Vlodavsky et al.** *Nature,* 1981, vol. 289, 304
- **Nakache et al.** *Nature,* 1985, vol. 317, 75
- **Jaye et al.** *Science,* 1985, vol. 228, 882
- **Montesano et al.** *Cell,* 1985, vol. 42, 469
- **Ensoli et al.** *Nature,* 1994, vol. 371, 674
- **Lu et al.** *J. Immunol.,* 1994, vol. 153, 1687
- **Juhasz et al.** *Am J Pathol,* 1993, vol. 143, 528
- **Rosenberg et al.** *N. Engl. J. Med.,* 1987, vol. 316, 889
- **Melder et al.** *Nat. Med.,* 1996, vol. 2, 992
- **Ferrara.** *Nat. Med.,* 1996, vol. 2, 971
- **Lippincott-Schwartz et al.** *Cell,* 1989, vol. 56, 801
- **Lippincott-Schwartz et al.** *Cell,* 1990, vol. 60, 821
- **Orci et al.** *Cell,* 1991, vol. 64, 1183
- **Kataoka et al.** *J. Immunol.,* 1996, vol. 156, 3678
- **Ohminami et al.** *Blood,* 1999, vol. 93, 925